(19) Europäisches Patentamt

European Patent Office

Office européen des brevets

(11) **EP 3 932 489 B1**

(12) **EUROPEAN PATENT SPECIFICATION**

(45) Date of publication and mention
of the grant of the patent:
**25.10.2023 Bulletin 2023/43**

(21) Application number: **20762445.3**

(22) Date of filing: **21.02.2020**

(51) International Patent Classification (IPC):
**A61K 9/70** *(2006.01)*    **A61P 29/00** *(2006.01)*
**A61K 47/20** *(2006.01)*    **A61K 47/32** *(2006.01)*
**A61K 31/196** *(2006.01)*

(52) Cooperative Patent Classification (CPC):
**A61K 9/7053; A61K 31/196; A61P 29/00**

(86) International application number:
**PCT/JP2020/007182**

(87) International publication number:
**WO 2020/175395 (03.09.2020 Gazette 2020/36)**

(54) **TRANSDERMAL PATCH**

TRANSDERMALES PFLASTER

TIMBRE TRANSDERMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.02.2019   JP 2019034363**

(43) Date of publication of application:
**05.01.2022 Bulletin 2022/01**

(73) Proprietor: **Hisamitsu Pharmaceutical Co., Inc.**
**Tosu-shi, Saga 841-0017 (JP)**

(72) Inventors:
• **KAMAKURA, Naoto**
**Tosu-shi, Saga 841-0017 (JP)**

• **TAKEUCHI, Akio**
**Tosu-shi, Saga 841-0017 (JP)**
• **KURITA, Hisakazu**
**Tosu-shi, Saga 841-0017 (JP)**

(74) Representative: **Grünecker Patent- und
Rechtsanwälte
PartG mbB
Leopoldstraße 4
80802 München (DE)**

(56) References cited:
**WO-A1-2013/191128    WO-A1-2018/070370
JP-A- 2007 015 963    JP-A- 2008 508 954**

## Description

### Technical Field

The present invention relates to a transdermal patch.

### Background Art

Dimethyl sulfoxide (DMSO) is an organic solvent having excellent drug solubility, and its use in transdermal patches is being studied. For example, Patent Literature 1 discloses a DMSO-containing transdermal patch that can suppress precipitation of crystals of high-concentration diclofenac and its sodium salt contained in an adhesive layer. In addition, Patent Literature 2 discloses a transdermal patch of which adhesiveness can be maintained even after being pasted for a long period of time by further combining with a backing having specific moisture permeability.

### Citation List

#### Patent Literature

[Patent Literature 1] WO2013/191128
[Patent Literature 2] WO2018/124089

### Summary of Invention

#### Technical Problem

However, the present inventors have found that if the transdermal patches disclosed in Patent Literatures 1 and 2 are pasted on a site (skin) that can extend and contract during motion, a part of each transdermal patch sometimes peels off.

Joints are portions where bones are connected to each other, and are mainly classified into diarthroses, amphiarthroses, and synarthroses. In addition, cartilage is present between bones and has functions of absorbing impacts and suppressing damage from rubbing between bones. Joint movements include, for example, flexion, extension, abduction, adduction, external rotation, internal rotation, pronation, supination, dorsiflexion, and plantar flexion. A diarthrosis is formed by combining a convex-shaped portion called a joint head with a concave-shaped portion called glenoid fossa. Examples of diarthroses include spherical joints (such as shoulder joints and hip joints), hinge joints (such as elbow joints, interphalangeal joints, and knee joints), rotary joints (such as humeroradial joints of the elbows and atlantoaxial joints of the neck), ellipsoid joints (such as hand joints), saddle joints (such as 1st carpometacarpal joints), and planar joints (such as ankle regions and carpal regions). An amphiarthrosis is a joint in which bones bind to each other by cartilage and which move within a range of several millimeters, and external impact is absorbed by the cartilage. Examples of amphiarthroses include the pubic symphysis and intervertebral discs of the spinal column. A synarthrosis is a joint in which bones are closely connected to each other and which do not move at all, and most of them are present in the skull.

The skin near a diarthrosis repeats extension and contraction in various directions according to movement of the joint mainly when the posture is changed during activities of daily living, exercise, or the like. The present inventors thought that, if the skin around an application site repeats extension and contraction, a part of a transdermal patch is likely to peel off due to stress applied to the transdermal patch itself. Therefore, an object of the present invention is to provide a DMSO-containing transdermal patch which is unlikely to peel off even in a case of being pasted on a site (skin) that can extend and contract during motion.

### Solution to Problem

The present inventors have found that, if a styrene-based thermoplastic elastomer having a specific triblock content is blended in an adhesive layer, a transdermal patch is unlikely to peel off even if it is pasted on a diarthrosis or bent portion.

That is, the present invention provides [1] to [6] below.

[1] A transdermal patch including: an adhesive layer on a backing, in which the adhesive layer contains a drug, DMSO, and an adhesive base, and the adhesive base contains a styrene-based thermoplastic elastomer having a triblock content of 35% to 65%.

[2] The transdermal patch according to [1], in which the adhesive layer has a viscosity of 450 Pa·s to 900 Pa·s at 60°C.

[3] The transdermal patch according to [1] or [2], in which the adhesive layer has a melt flow rate of 1.1 to 2 g/10 min at 60°C.

[4] The transdermal patch according to any of [1] to [3], in which the adhesive layer has tan δ of 0.53 to 0.8 at 1 Hz.

[5] The transdermal patch according to any of [1] to [4], which is pasted on skin that can extend and contract during motion.

[6] A method for producing a transdermal patch including an adhesive layer on a backing and a release liner on an opposite surface to a surface coming into contact with the backing of the adhesive layer, the method comprising:

Step A of mixing a drug, DMSO, and an adhesive base containing a styrene-based thermoplastic elastomer with each other to obtain an adhesive composition;

Step B of stirring the adhesive composition using a mixer in a nitrogen stream so that the styrene-based thermoplastic elastomer has a triblock content of 35% to 65%; and

a step of spreading the adhesive composition obtained in Step B on the release liner to stack the backing on the spread adhesive composition.

**Advantageous Effects of Invention**

According to the present invention, it is possible to provide a DMSO-containing transdermal patch which is unlikely to peel off even in a case of being pasted on a site (skin) that can extend and contract during motion. A site that can extend and contract during motion is, for example, skin that extends and contracts according to the movement of a diarthrosis (particularly a shoulder joint, a hip joint, an elbow joint, an interphalangeal joint, a knee joint, a humeroradial joint of an elbow, an atlantoaxial joint of the neck, a hand joint, a 1st carpometacarpal joint, an ankle region, or a carpal region), and examples thereof include the neck, the shoulders, the upper back, the elbows, the wrists, the waist, the lower abdomen, the knees, and the ankles.

The transdermal patch according to the present invention has superior cohesive force so that the residue of plaster during adhesion or when releasing the transdermal patch can be reduced and clothes or the like can be kept clean without contamination.

**Brief Description of Drawings**

The transdermal patch of the present invention includes: an adhesive layer on a backing. The adhesive layer is usually stacked on one surface of a backing, and a peelable film is stacked on the other surface of the adhesive layer as necessary.

Any backing that can hold an adhesive layer may be used, and those common in the technical field can be used. Examples of backing include woven fabrics (including knitted fabrics), non-woven fabrics, and non-porous or porous films (sheets). The backing may be a stacked body of a woven fabric and a film or a stacked body of a non-woven fabric and a film.

The material of the backing is preferably one or more materials selected from polyesters (such as polyethylene terephthalate (PET), polyethylene isophthalate, polypropylene terephthalate, polypropylene isophthalate, polybutylene terephthalate, and polyethylene naphthalate), polyolefins (polymers or copolymers of vinyl monomers such as ethylene, propylene, vinyl acetate, or acrylonitrile), polyamides (nylon or silk), polyurethanes (PU), or cellulose (such as cotton or hemp).

The thickness of the backing is, for example, 0.1 mm to 2 mm. The basis weight of the backing is, for example, 30 g/m$^2$ to 200 g/m$^2$. In the present specification, the thickness and the basis weight of the backing are measured according to the JIS L 1906:2000 standard.

In a case where the backing is a woven fabric or a non-woven fabric, the backing may be further coated with a rubber composition. Rubber compositions include polyisoprene, polyisobutylene (PIB), polybutadiene, a styrene-butadiene-styrene block copolymer, a styrene-isoprene-styrene (SIS) block copolymer, styrene-butadiene rubber, styrene-isoprene rubber, and combinations thereof. In addition, the rubber composition may further contain additives such as a tackifier, a plasticizer, or a filler. The tackifier is, for example, an alicyclic saturated hydrocarbon resin, a hydrogenated rosin ester, a terpene resin, or a combination thereof.

In a case where the backing is a knitted fabric, the 50% modulus (JIS L 1018:1999) of the backing in either the longitudinal direction (material flow direction) or the lateral direction (material width direction) is also preferably 1 N/50 mm to 12 N/50 mm. In a case where the 50% modulus is less than or equal to 12 N/50 mm, the amount of stress applied to the transdermal patch due to expansion and contraction of the skin is smaller, and therefore the adhesion to the skin is more favorable.

In a case where the backing is a film, the material thereof is preferably one such as a polyurethane having high moisture permeability (high DMSO permeability). Since a film made of a polyurethane has excellent stretchability, it is preferable from the viewpoint of enhancing adhesion of the transdermal patch to the skin and stretch followability of the transdermal patch.

The backing is preferably, for example, a film or non-woven fabric made of a polyurethane, a knitted fabric made of polyethylene terephthalate, a polyester cloth coated with a rubber composition, or a combination thereof. More specifically, the backing is preferably a stacked body of a film made of polyurethane and a non-woven fabric made of a polyurethane fiber, a non-woven fabric made of a PET fiber, or a polyester cloth coated with a rubber composition.

The moisture permeability of the backing is preferably greater than or equal to 400 g/m$^2$·24 hours or more. If a backing having such high moisture permeability is used, DMSO gradually volatilizes from the transdermal patch applied to the skin. Therefore, the adhesiveness of the transdermal patch is more improved and the transdermal patch is unlikely to peel off even if it is applied for a long period of time. The moisture permeability is originally an indicator of water permeability, but the present inventors have found that a backing with high moisture permeability has high DMSO permeability (volatility). The moisture permeability of the backing may be, for example, greater than or equal to 422 g/m$^2$·24 hours, greater than or equal to 750 g/m$^2$·24 hours, greater than or equal to 2,000 g/m$^2$·24 hours, greater than or equal to 2,077 g/m$^2$·24 hours, greater than or equal to 4,000 g/m$^2$·24 hours, greater than or equal to 5,500 g/m$^2$·24 hours, greater than or equal to 5,667 g/m$^2$·24 hours, or greater than or equal to 8,408 g/m$^2$·24 hours. The upper limit value of the moisture permeability may be 20,000 g/m$^2$·24 hours. If the moisture permeability of the backing is within such a range, DMSO more easily volatilizes from the adhesive layer, and therefore the backing is more effective for improving the adhesiveness of the transdermal patch.

In the present specification, the moisture permeability of the backing is moisture permeability at 40°C which is defined according to the JIS Z 0208:1976 standard (moisture permeability test method (cup method) of the moisture-proof packaging material).

The adhesive layer is a portion press-bonded to the skin when a transdermal patch is applied, and contains at least a drug, DMSO, and an adhesive base. In addition, the adhesive base contains at least a styrene-based thermoplastic elastomer.

The drug is not particularly limited as long as it is soluble in DMSO and can be administered transdermally. Examples of drugs include anti-inflammatory analgesics such as loxoprofen, felbinac, flurbiprofen, indomethacin, ketoprofen, and diclofenac, narcotic analgesics such as buprenorphine, fentanyl, and butorphanol, clonidine, estradiol, tulobuterol, and oxybutynin. The drug may be a free form, pharmaceutically acceptable salts thereof, or a mixture thereof.

In the present specification, examples of "pharmaceutically acceptable salts" include inorganic acid salts such as hydrochlorides, nitrates, and phosphates, organic acid salts such as acetates, lactates and benzenesulfonates, and inorganic base salts such as sodium salts, potassium salts, and calcium salts. More specific examples of pharmaceutically acceptable salts include loxoprofen sodium, diclofenac sodium, fentanyl citrate, butorphanol tartrate, clonidine hydrochloride, tulobuterol hydrochloride, and oxybutynin hydrochloride.

The content of a drug is, for example, 1 to 30 mass%, 2 to 20 mass%, or 3 to 10 mass% based on the total mass of an adhesive layer. A drug contained in the form of pharmaceutically acceptable salts is converted to a free form thereof.

DMSO dissolves a drug to improve the skin permeability of the drug. On the other hand, DMSO causes a decrease in adhesiveness of an adhesive layer. From the viewpoint of balancing these, the content of DMSO based on the total mass of an adhesive layer is preferably 1 to 20 mass%, more preferably 1 to 15 mass%, still more preferably 2 to 10 mass%, and particularly preferably 3 to 10 mass%.

The content of DMSO is preferably 1:0.1 to 1:5 based on the mass of a drug, for example. In particular, in a case where the drug is diclofenac sodium, the content of DMSO based on diclofenac sodium is preferably 1:0.3 to 1:4 and more preferably 1:0.4 to 1:3, 1:0.6 to 1:3, or 1:0.72 to 1:3. If the content of DMSO is within the above-described ranges, the skin permeability of diclofenac sodium is further improved, and crystals of diclofenac sodium are less likely to precipitate.

The adhesive base may contain a styrene-based thermoplastic elastomer, and those generally used in transdermal patches can be used. The adhesive base may further contain a rubber adhesive base, an acrylic adhesive base, or a silicone adhesive base in addition to a styrene-based thermoplastic elastomer.

The styrene-based thermoplastic elastomer is an elastomer exhibiting plasticity when heated, and has a styrene structure as a monomer unit. Examples of such styrene-based thermoplastic elastomers include a styrene-isoprene-styrene (SIS) triblock copolymer, a styrene-isoprene (SI) diblock copolymer, a styrene-butadiene-styrene triblock copolymer, a styrene-butadiene diblock copolymer, styrene-butadiene rubber, or styrene-isoprene rubber.

The styrene-based thermoplastic elastomer contains a triblock copolymer. A preferred triblock copolymer is an SIS triblock copolymer. The styrene-based thermoplastic elastomer may be a mixture of an SIS triblock copolymer and an SI diblock copolymer.

The styrene-based thermoplastic elastomer has a predetermined triblock content. The triblock content may be 35% to 65% and preferably 43% to 58%, 43.54% to 57.44%, or 50.71% to 57.44%. If the triblock content is greater than or equal to 35%, the residue of plaster of an adhesive layer is unlikely to be generated. If the triblock content is less than or equal to 65%, the adhesiveness tends to be further improved.

The "triblock content" in the present specification means a ratio of a triblock copolymer to the total amount of styrene-based thermoplastic elastomer contained in an adhesive layer. The triblock content can be calculated using the following equation from a proportion of a peak area value of each styrene-based thermoplastic elastomer in a chromatogram obtained when peaks are detected with a differential refractometer by dissolving an adhesive layer in an organic solvent and subjecting the total amount of the resultant to gel filtration chromatography.

$$\text{Triblock content [\%]} = (\text{peak area value of triblock copolymer}) /$$

$$(\text{total peak area value of styrene-based thermoplastic elastomers}) \times 100$$

In order to keep the triblock content of a styrene-based thermoplastic elastomer within a desired range, the content of a triblock copolymer and another styrene-based thermoplastic elastomer may be appropriately adjusted for mixing, or a triblock copolymer may be modified. Examples of methods for modifying a triblock copolymer include a method for performing stirring using a mixer in a nitrogen stream. By adjusting the nitrogen flow rate, the temperature of a mixer, and the speed or the time of stirring, a triblock copolymer can be modified to obtain a desired triblock content.

A rubber adhesive base other than a styrene-based thermoplastic elastomer may be a polymer mainly composed of natural or synthetic rubber. Examples of such rubber adhesive bases include polyisoprene, polyisobutylene (PIB), and polybutadiene. In particular, if an SIS block copolymer and PIB are combined, the skin permeability of a drug tends to further increase and the adhesiveness of a transdermal patch also tends to further increase.

In a case where the adhesive base is a rubber adhesive base, the content of the adhesive base based on the total mass of an adhesive layer may be 10 to 70 mass%, 10 to 40 mass%, 15 to 50 mass%, 15 to 35 mass%, or 30 to 40 mass% from the viewpoint of the adhesiveness of the transdermal patch.

The acrylic adhesive base is a polymer or a copolymer containing (meth)acrylic acid or an ester thereof as a monomer unit. The acrylic adhesive base is, for example, an adhesive obtained by polymerizing or copolymerizing at least one of (meth)acrylic monomers such as (meth)acrylic acid, 2-ethylhexyl (meth)acrylate, methyl (meth)acrylate, butyl (meth)acrylate, and hydroxyethyl (meth)acrylate. Examples of acrylic adhesive bases include poly(methyl acrylate/2-ethylhexyl acrylate) and poly(methacrylic acid/n-butyl acrylate).

The content of the adhesive base in a case where the adhesive base is an acrylic adhesive base or a silicone adhesive base may be 50 to 90 mass% with respect to the total mass of the adhesive layer.

In the present specification, "(meth)acrylic acid" and similar terms mean acrylic acid, methacrylic acid, or both.

The silicone adhesive base contains, for example, a silicone rubber such as polydimethylpolysiloxane, polymethylvinylsiloxane, or polymethylphenylsiloxane as a main component.

The adhesive layer may further contain other components (such as a tackifier, a plasticizer, a filler, a stabilizer, a transdermal absorption promoter of a drug, a fragrance, or a colorant).

Examples of tackifiers include a terpene resin, a terpene phenol resin, a rosin ester resin, a hydrogenated rosin ester resin, an alicyclic saturated hydrocarbon resin, and a petroleum-based resin. The terpene resin is preferably a hydrogenated terpene resin. Examples of terpene resins include an $\alpha$-pinene resin, a $\beta$-pinene resin, an aromatic modified terpene resin, and a terpene phenol resin. Preferred tackifiers are a hydrogenated rosin ester resin, an alicyclic saturated hydrocarbon resin, and a combination thereof.

Examples of plasticizers include paraffin oils (such as liquid paraffin), squalane, squalene, vegetable oils (such as olive oil, camellia oil, castor oil, tall oil, peanut oil, spearmint oil, eucalyptus oil, jojoba oil, camphor white oil, sunflower oil, and orange oil), oils and fats (such as dibutyl phthalate and dioctyl phthalate), and liquid rubber (such as liquid polybutene and liquid isoprene rubber).

Examples of fillers include powders of metal compounds (such as aluminum oxide, aluminum hydroxide, zinc oxide, titanium oxide, and calcium carbonate), ceramics (such as talc, clay, kaolin, silica, hydroxyapatite, synthetic aluminum silicate, and magnesium aluminometasilicate), or organic compounds (such as cellulose powder and stearate), or short fibers of resins containing the powders.

The transdermal absorption promoter may be a compound known to have a transdermal absorption promoting action on the skin in the related art. Examples of transdermal absorption promoters include organic acids and salts thereof (for example, aliphatic carboxylic acids (hereinafter, also referred to as "fatty acids") having 6 to 20 carbon atoms) and salts thereof, cinnamic acid and salts thereof), organic acid esters (for example, fatty acid esters and cinnamic acid esters), organic acid amides (for example, fatty acid amides), fatty alcohols, polyhydric alcohols, and ethers (for example, fatty ethers and polyoxyethylene alkyl ethers). These absorption promoters may have unsaturated bonds, or may have a cyclic, linear, or branched chemical structure. In addition, the transdermal absorption promoter may be a monoterpene compound, a sesquiterpene compound, or a vegetable oil (for example, olive oil). These transdermal absorption promoters may be used alone or in a combination of two or more thereof.

Examples of such organic acids include aliphatic (mono-, di-, or tri-)carboxylic acids (for example, acetic acid, propionic acid, citric acid (including anhydrous citric acid), isobutyric acid, caproic acid, caprylic acid, fatty acids, lactic acid, maleic acid, pyruvic acid, oxalic acid, succinic acid, and tartaric acid), aromatic carboxylic acids (for example, phthalic acid, salicylic acid, benzoic acid, and acetylsalicylic acid), cinnamic acid, alkanesulfonic acids (for example, methanesulfonic acid, ethanesulfonic acid, propanesulfonic acid, and butanesulfonic acid), alkanesulfonic acid derivatives (for example, polyoxyethylene alkyl ether sulfonic acids and N-2-hydroxyethylpiperazine-N'-2-ethanesulfonic acid), and cholic acid derivatives (for example, dehydrocholic acid). These organic acids may be alkali metal salts such as sodium salts. Among these, aliphatic carboxylic acids, aromatic carboxylic acids, or salts thereof are preferable, and acetic acid, sodium acetate, or citric acid is particularly preferable.

Examples of fatty acids include lauric acid, myristic acid, palmitic acid, stearic acid, isostearic acid, oleic acid, linoleic acid, and linolenic acid.

Examples of organic acid esters include ethyl acetate, propyl acetate, cetyl lactate, lauryl lactate, methyl salicylate, ethylene glycol salicylate, methyl cinnamate, and fatty acid esters. Examples of fatty acid esters include methyl laurate, hexyl laurate, isopropyl myristate, myristyl myristate, octyldodecyl myristate, and cetyl palmitate. Fatty acid esters may be glycerin fatty acid esters, propylene glycol fatty acid esters, sorbitan fatty acid esters, polyethylene glycol sorbitan fatty acid esters, polyethylene glycol fatty acid esters, sucrose fatty acid esters, or polyoxyethylene hydrogenated castor oil. Specific examples of fatty acid esters include glycerin monocaprylate, glycerin monocaprate, glycerin monolaurate, glycerin monooleate, sorbitan monolaurate, sucrose monolaurate, polysorbate 20 (trade name), propylene glycol monolaurate, polyethylene glycol monolaurate, polyethylene glycol monostearate, Span 20, Span 40, Span 60, Span 80, Span 120 (trade names), Tween 20, Tween 21, Tween 40, Tween 60, Tween 80 (trade names), and NIKKOL HCO-60 (trade name).

Examples of organic acid amides include fatty acid amides (for example, lauric acid diethanolamide), hexahydro-1-dodecyl-2H-azepine-2-one (also referred to as Azone), and derivatives thereof, and pyrothiodecane.

Fatty alcohols are aliphatic alcohols having 6 to 20 carbon atoms. Examples of fatty alcohols include lauryl alcohol, myristyl alcohol, oleyl alcohol, isostearyl alcohol, and cetyl alcohol. Examples of polyhydric alcohols include propylene glycol.

Examples of polyoxyethylene alkyl ethers include polyoxyethylene lauryl ethers.

Examples of monoterpene compounds include geraniol, thymol, eugenol, terpineol, l-menthol, borneol, d-limonene, isoeugenol, isoborneol, nerol, and dl-camphor.

Oleyl alcohol, lauryl alcohol, isostearyl alcohol, lauric acid diethanolamide, glycerin monocaprylate, glycerin monocaprate, glycerin monooleate, sorbitan monolaurate, propylene glycol monolaurate, a polyoxyethylene lauryl ether, or pyrothiodecane is more preferable. Fatty acids are preferable, and oleic acid is particularly preferable.

The viscosity of the adhesive layer when measured at 60°C is preferably 450 to 900 Pa·s and more preferably 500 to 700 Pa·s. If the viscosity is within a range of 450 to 900 Pa·s, more favorable adhesiveness is exhibited. If the viscosity is less than 450 Pa·s, the adhesive layer is likely to bite into a backing, and the adhesive strength tends to decrease. If the viscosity exceeds 900 Pa·s, the adhesive layer becomes hard, and the adhesive strength tends to decrease. The method for measuring the viscosity will be described below.

The melt flow rate of the adhesive layer when measured at 60°C is preferably 1.1 to 2 g/10 min and more preferably 1.2 to 1.8 g/10 min. If the melt flow rate is within a range of 1.1 to 2 g/10 min, more favorable adhesiveness is exhibited. If the melt flow rate is less than 1.1 g/10 min, the adhesive layer is likely to bite into a backing, and the adhesive strength tends to decrease. If the melt flow rate exceeds 2 g/10 min, the fluidity of the adhesive layer deteriorates, and a transdermal patch tends to be likely to peel off when pasted on a diarthrosis or a bent portion. The method for measuring the melt flow rate will be described below.

In the adhesive layer, the loss tangent (tan δ) in dynamic viscoelasticity measurement at a frequency of 1 Hz at 32°C is preferably 0.53 to 0.8 and more preferably 0.55 to 0.75. If the loss tangent (tan δ) is within a range of 0.53 to 0.8, more favorable adhesiveness is exhibited. If the loss tangent (tan δ) is less than 0.53, the elasticity of the adhesive layer increases, and a transdermal patch tends to be likely to peel off when pasted on a diarthrosis or a bent portion. If the loss tangent (tan δ) exceeds 0.8, the adhesive layer is likely to bite into a backing, and the adhesive strength tends to decrease. In the present specification, the loss tangent (tan δ) is a value which is calculated using the following equation such that a composition containing a drug, DMSO, and an adhesive base is sandwiched between two plates, and the change in stress when vibration is periodically applied to one plate to give distortion is measured in the dynamic viscoelasticity measurement. The dynamic viscoelasticity measurement is performed using a rotary rheometer at a temperature of 32°C and a frequency of 1 Hz, for example.

$$\text{Loss tangent (tan }\delta) = \text{loss modulus (G'') / storage modulus (G')}$$

The adhesive layer may include a release liner, which is on an opposite surface to a backing, on the surface coming into contact with the skin. The release liner is a liner which is removed when a transdermal patch is used, and is not limited as long as it is generally used in a transdermal patch. Examples of the material of the release liner include polyester (such as polyethylene terephthalate (PET)), polyolefin (such as polypropylene and polyethylene), and cellulose compounds (such as paper). The release liner may have a sheet shape consisting of a stacked body of the above-described material. The surface of the release liner is preferably subjected to release treatment with silicone, fluorinated polyolefin, or the like.

In the present specification, being "unlikely to peel off even if it is pasted on diarthroses or bent portions" means that, in a case where a transdermal patch cut into a rectangle (20 mm × 70 mm) is pasted on a rubber sheet (adherend) and press-bonded thereto, and is then fixed along a curved surface portion of a cylindrical object having a predetermined diameter, the length (the distance from an end portion of an adhesive layer which first peels off) of a peeled portion is shorter than or equal to 30 mm and a probe tack value is greater than or equal to 0.75 N. The shorter the length of the peeled portion or the higher the probe tack value, the better the effect. Such evaluation can be determined using results of tests conducted with reference to the descriptions of Test Examples 3 and 4.

The transdermal patch can be produced, for example, through the following method. However, the present invention is not limited thereto and the transdermal patch can be produced through known methods. First, components constituting an adhesive layer are mixed with each other at predetermined proportions to obtain a uniform composition. Next, the obtained composition is spread on a release liner to a predetermined thickness to form an adhesive layer. Next, a backing is press-bonded to the adhesive layer so that the adhesive layer is sandwiched between the release liner and the backing. Finally, the transdermal patch can be obtained by cutting the stacked body into a predetermined shape. In this case, the release liner is removed at the time of applying the transdermal patch.

**Examples**

Hereinafter, the transdermal patch of the present invention will be described in more detail using examples and comparative examples.

(Preparation of Transdermal Patch)

An SIS triblock copolymer, polyisobutylene, and liquid paraffin were weighed in the amounts shown in Table 1 and mixed with each other with a mixer in a nitrogen stream, and remaining components were added thereto to obtain a composition. Through the mixing process with a mixer, the SIS triblock copolymer was modified into a mixture (styrene-based thermoplastic elastomer) of the SIS triblock copolymer and an SI diblock copolymer. The obtained composition was uniformly spread on a release liner (a PET film to which release treatment was subjected) to form an adhesive layer (thickness of 214 $\mu$m, unit mass of 214 $g/m^2$). A non-woven fabric (backing) made of polyethylene terephthalate was stacked on the adhesive layer to obtain a transdermal patch. In Table 1, each styrene-based thermoplastic elastomer is one prepared by modifying a commercially available SIS triblock copolymer under different conditions. Transdermal patches of Comparative Examples 1 and 2 respectively correspond to transdermal patches of Patent Literatures 1 and 2.

(Measurement of Triblock Content)

Each adhesive layer of the transdermal patches was dissolved in tetrahydrofuran, and an insoluble substance was filtered to obtain a sample solution. The obtained sample solution was subjected to gel filtration chromatography and subjected to separation under the following conditions, and the peak areas of the SIS triblock copolymer and the SI diblock copolymer were measured. The triblock content (SIS content) was calculated using the following equation.

$$\text{Triblock content (\%)} = (\text{peak area value of SIS}) / (\text{peak area value of SIS} + \text{peak area value of SI}) \times 100$$

<Analysis Conditions>

Device name: Gel Permeation Chromatography System (manufactured by Shimadzu Corporation)
Column: TSKgel G500011XL
Column temperature: 40°C

Detector: Differential refractometer (response time: 500 msec)
Flow rate: 1 mL/min
Injection amount: 100 μL
Mobile phase: Tetrahydrofuran

The calculated triblock contents are shown in Table 1.

[Table 1]

| | Comp. Ex. 1 | Ex. 1 | Ex. 2 | Comp. Ex. 2 |
|---|---|---|---|---|
| Diclofenac sodium | 5.00 | 5.00 | 5.00 | 5.00 |
| DMSO | 7.00 | 7.00 | 7.00 | 7.00 |
| Styrene-based thermoplastic elastomer (1) | 19.15 | - | - | - |
| Styrene-based thermoplastic elastomer (2) | - | 19.15 | - | - |
| Styrene-based thermoplastic elastomer (3) | - | - | 19.15 | - |
| Styrene-based thermoplastic elastomer (4) | - | - | - | 19.15 |
| Polyisobutylene | 8.21 | 8.21 | 8.21 | 8.21 |
| Tackifier | 37.00 | 37.00 | 37.00 | 37.00 |
| Liquid paraffin | 12.53 | 12.53 | 12.53 | 12.53 |
| Other components | 11.11 | 11.11 | 11.11 | 11.11 |
| Total | 100 | 100 | 100 | 100 |
| Triblock content [%] | 69.4 | 57.44 | 50.71 | 30.13 |

Test Example 1: Measurement of Viscosity and Melt Flow Rate

About 1 g of each adhesive layer of the transdermal patches was weighed, and the viscosity (unit: Pa·s) and the melt flow rate (unit: g/10 min) were measured using a flow tester (manufactured by Shimadzu Corporation) under the conditions of a die hole diameter of 0.5 mm, a die length of 1 mm, and a temperature of 60°C.

Test Example 2: Measurement of Dynamic Viscoelasticity tan δ

About 130 mg of each adhesive layer of the transdermal patches was weighed, and the dynamic viscoelasticity tan δ was measured while changing a frequency between 0.01 to 80 Hz using the rheometer (manufactured by Thermo Fisher Scientific Inc.) under the conditions of a plate size of 8 mm, a distortion of 1%, a temperature of 32°C, and a gap of 1 mm.

Test Example 3: Repulsion Resistance Test

Each transdermal patch was cut into a rectangle (20 mm × 70 mm), and a polyethylene terephthalate (PET) film (thickness of 250 μm, 20 mm × 70 mm rectangle) was pasted on the backing side with a double-sided adhesive tape. Thereafter, the release liner of the transdermal patch was removed and pasted on a rubber sheet (adherend) and press-bonded by rolling a roller with a mass of 1.5 kg back and forth once to obtain a stacked body of the PET film, the transdermal patch, and the rubber sheet. The obtained stacked body was fixed along a curved surface portion of a cylindrical object having a diameter of 30 mm. One minute after the fixation, the distance (the distance from an end portion of an adhesive layer which first peels off, unit: mm) of a peeled portion was measured.

Test Example 4: Measurement of Probe Tack Value

Each transdermal patch was punched into a circle (diameter of 22 mm), and a probe tack value (unit: N) of each adhesive layer was measured with a probe tack tester (manufactured by Tester Sangyo Co., Ltd.) using a stainless steel probe under the conditions of a speed of 1 cm/sec and a contact time of 1 second.

Test Example 5: 90° Peel Measurement Test

Each transdermal patch was cut into a rectangle (40 mm × 20 mm), and each release liner was removed. A short side end of rectangular (60 mm × 30 mm) paper was pasted on the exposed adhesive layer at a position 5 mm from a long side end, and was then pasted on a polytetrafluoroethylene (PTFE) plate and press-bonded by rolling a rubber roller with a mass of 2 kg back and force twice at a speed of 3,000 mm/min. That is, a part of the paper was sandwiched between the adhesive layer and the PTFE plate, and the remaining part of the paper protruded to the outside of the adhesive layer.

The protruding portion of the paper was folded back so as to be 90° with respect to the adhered surface of the PTFE plate and set in an autograph device (manufactured by Shimadzu Corporation). By pulling the paper at a speed of 300 mm/min, the presence or absence of the residue of plaster when the adhesive layer peeled off from the PTFE plate was observed.

The results of Test Examples 1 to 5 are shown in Table 2. In the transdermal patches of Examples 1 and 2, the results of Test Examples 1 to 4, particularly Test Examples 3 and 4 were better than those of the transdermal patches of Comparative Examples 1 and 2. In particular, it was suggested that the transdermal patches of Examples 1 and 2 were unlikely to peel off even if these were pasted on diarthroses or bent portions.

[Table 2]

|  |  | Comp. Ex. 1 | Ex. 1 | Ex. 2 | Comp. Ex. 2 |
|---|---|---|---|---|---|
| Test Example 1 | Viscosity [Pa·s] | 963 | 687 | 642 | 392 |
|  | Melt flow rate [g/10 min] | 0.944 | 1.316 | 1.406 | 2.334 |
| Test Example 2 | Dynamic viscoelasticity | 0.517 | 0.59 | 0.646 | 0.855 |
| Test Example 3 | Distance of peeled portion [mm] | 43 | 22.2 | 22.8 | 24.7 |
| Test Example 4 | Probe tack [N] | 0.7 | 1.07 | 0.99 | 0.69 |
| Test Example 5 | Residue of plaster | None | None | None | None |

(Preparation of Transdermal Patch)

An SIS triblock copolymer, polyisobutylene, and liquid paraffin were weighed in the amounts shown in Table 1 and mixed with each other with a mixer in a nitrogen stream, and remaining components were added thereto to obtain a composition. Through the mixing process with a mixer, the SIS triblock copolymer was modified into a mixture (styrene-based thermoplastic elastomer) of the SIS triblock copolymer and an SI diblock copolymer. The obtained composition was uniformly spread on a release liner (a PET film to which release treatment was subjected) to form an adhesive layer (thickness of 214 $\mu$m, unit mass of 214 g/m$^2$). A non-woven fabric (backing) made of polyethylene terephthalate was stacked on the adhesive layer to obtain a transdermal patch. In Table 3, each styrene-based thermoplastic elastomer is one prepared by modifying a commercially available SIS triblock copolymer under different conditions.

[Table 3]

|  | Ex. 3 | Ex. 4 | Ex. 5 |
|---|---|---|---|
| Diclofenac sodium | 5.00 | 5.00 | 5.00 |
| DMSO | 7.00 | 7.00 | 7.00 |
| Styrene-based thermoplastic elastomer (5) | 19.15 | - | - |
| Styrene-based thermoplastic elastomer (6) | - | 19.15 | - |
| Styrene-based thermoplastic elastomer (7) | - | - | 19.15 |
| Polyisobutylene | 8.21 | 8.21 | 8.21 |
| Alicyclic saturated hydrocarbon resin | 27.0 | 27.0 | 27.0 |
| Hydrogenated rosin glycerin ester | 10.0 | - | 10.0 |
| Terpene resin | - | 10.0 | - |
| Liquid paraffin | 12.53 | 12.53 | 12.53 |

(continued)

|  | Ex. 3 | Ex. 4 | Ex. 5 |
|---|---|---|---|
| Other components | 11.11 | 11.11 | 11.11 |
| Total | 100 | 100 | 100 |
| Triblock content [%] | 39.4 | 44.0 | 62.8 |

Regarding the obtained transdermal patches, the results in which Test Examples 1 to 5 are carried out are shown in Table 4. In the transdermal patches of Examples 3 to 5, the results of Test Examples 1 to 4 were favorable. In particular, it was suggested that the transdermal patch of Example 5 was unlikely to peel off even if it was pasted on diarthroses or bent portions.

[Table 4]

|  |  | Ex. 3 | Ex. 4 | Ex. 5 |
|---|---|---|---|---|
| Test Example 1 | Viscosity [Pa·s] | 553 | 792 | 665 |
|  | Melt flow rate [g/10 min] | 1.64 | 1.14 | 1.36 |
| Test Example 2 | Dynamic viscoelasticity | 0.74 | 0.91 | 0.59 |
| Test Example 3 | Distance of peeled portion [mm] | 22.3 | 25.5 | 23.1 |
| Test Example 4 | Probe tack [N] | 0.75 | 0.98 | 1.05 |
| Test Example 5 | Residue of plaster | None | None | None |

**Claims**

1. A transdermal patch comprising an adhesive layer on a backing,

   wherein the adhesive layer comprises a drug, DMSO, and an adhesive base, and
   wherein the adhesive base comprises a styrene-based thermoplastic elastomer having a triblock content of 35% to 65%.

2. The transdermal patch according to claim 1,
   wherein the adhesive layer has a viscosity of 450 Pa·s to 900 Pa·s at 60°C, measured as disclosed in the description.

3. The transdermal patch according to claim 1 or 2,
   wherein the adhesive layer has a melt flow rate of 1.1 to 2 g/10 min at 60°C, measured as disclosed in the description.

4. The transdermal patch according to any one of claims 1 to 3,
   wherein the adhesive layer has a loss tangent of 0.53 to 0.8 at 1 Hz, measured as disclosed in the description.

5. The transdermal patch according to any one of claims 1 to 4, which is applied to skin that can extend and contract during motion.

**Patentansprüche**

1. Transdermales Pflaster, das eine Klebeschicht auf einem Trägermaterial umfasst,

   wobei die Klebeschicht ein Medikament (DMSO) sowie eine Klebstoffbasis umfasst, und
   wobei die Klebstoffbasis ein thermoplastisches Elastomer auf Styrol-Basis mit einem Triblock-Gehalt von 35 % bis 65 % umfasst.

2. Transdermales Pflaster nach Anspruch 1,
   wobei die Klebeschicht eine Viskosität von 450 Pa·s bis 900 Pa·s bei 60 °C, gemessen wie in der Beschreibung

**EP 3 932 489 B1**

offenbart, hat.

3. Transdermales Pflaster nach Anspruch 1 oder 2,
wobei die Klebstoffschicht eine Schmelze-Massefließrate von 1,1 bis 2 g/10 min bei 60 °C, gemessen wie in der Beschreibung offenbart, hat.

4. Transdermales Pflaster nach einem der Ansprüche 1 bis 3,
wobei die Klebstoffschicht einen Verlustfaktor von 0,53 bis 0,8 bei 1 $H_2$, gemessen wie in der Beschreibung offenbart, aufweist.

5. Transdermales Pflaster nach einem der Ansprüche 1 bis 4, das auf Haut aufgebracht wird, die sich bei Bewegung ausdehnen und zusammenziehen kann.


**Revendications**

1. Timbre transdermique comprenant une couche adhésive sur un support, dans lequel la couche adhésive comprend un médicament, du DMSO et une base adhésive, et
dans lequel la base adhésive comprend un élastomère thénoplastique à base de styrène ayant une teneur en triblocs de 35 % à 65 %.

2. Timbre transdermique selon la revendication 1, dans lequel la couche adhésive a une viscosité comprise entre 450 Pa.s et 900 Pa.s à 60°C, mesurée comme indiqué dans la description.

3. Timbre transdermique selon les revendications 1 ou 2, dans lequel la couche adhésive a un taux de fluidité compris entre 1,1 et 2 g/10 min à 60°C, mesuré comme indiqué dans la description.

4. Timbre transdermique selon l'une des revendications 1 à 3, dans lequel la couche adhésive a une tangente de perte comprise entre 0,53 et 0,8 à 1 $H_2$, mesurée comme indiqué dans la description.

5. Timbre transdermique selon l'une des revendications 1 à 4, qui est appliqué sur une peau qui peut s'étendre et se contracter pendant le mouvement.

**EP 3 932 489 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013191128 A

- WO 2018124089 A